# EUROPEAN PATENT SPECIFICATION

(11) **EP 0 568 027 B1**
(45) Date of publication and mention of the grant of the patent: **02.12.1998**
(21) Application number: 93106840.7
(22) Date of filing: 27.04.1993
(51) Int. Cl.: A01N 63/00, C12N 1/20

(54) **Method for producing a microorganism which is a natural enemy of nematodes**
Verfahren zur Herstellung von einem Mikro-Organismus, der ein natürlicher Feind von Nematoden ist
Méthode pour produire un micro-organisme qui est un ennemi naturel des nématodes

(30) Priority: 28.04.1992 JP 134454/92
(43) Date of publication of application: 03.11.1993
(73) Proprietor: NEMATECH CO., LTD., Minato-ku, Tokyo (JP)
(72) Inventor: Kasumimoto, Takanori, c/o Nematech Co., Ltd., Ibaraki-ken (JP); Kawada, Hiroshi, c/o Nematech Co., Ltd., Ibaraki-ken (JP); Ikeda, Ruriko, c/o Nematech Co., Ltd., Ibaraki-ken (JP)
(74) Representative: Wächtershäuser, Günter, Prof. Dr.

(56) References cited:
- EP-A- 0 217 378
- EP-A- 0 406 103
- EP-A- 0 492 946
- REVUE DE NEMATOLOGIE vol. 14, no. 4, 1991, BONDY FR pages 611 - 618 DAVIES K G; LAIRD V; KERRY B R 'THE MOTILITY DEVELOPMENT AND INFECTION OF MELOIDOGYNE-INCOGNITA ENCUMBERED WITH SPORES OF THE OBLIGATE HYPERPARASITE PASTEURIA-PENETRANS'
- DATABASE WPI Week 8644, Derwent Publications Ltd., London, GB; AN 86-287925 Ä44Ü
- DATABASE WPI Week 8312, Derwent Publications Ltd., London, GB; AN 83-28348K Ä12Ü

## Description

The present invention relates to a method for producing an obligate parasitic microorganism which is natural enemy of plant parasitic nematodes which causes damage to crop plants.

To avoid damage caused by nematodes, it has been common to employ a chemical control method or a field husbandry control method. From an economical background such that profitable crop plants have to be cultivated by continuous cropping, a convenient inexpensive chemical control method has been widely used. However, in view of a concern about environmental pollution due to treatment with a large amount of a chemical agent, a study is also being made on a method of employing a natural enemy microorganism as one of new control methods. Attention has been drawn particularly to Pasteuria spp. i.e. a group of bacteria which are obligate parasites of nematodes, since they are parasitic to certain specific nematodes and have relatively high resistance against heat and chemical substances and thus can be stored for long periods of time, and thus are highly safe and easy to use. However, it has been extremely difficult to parasitize them efficiently on nematodes and multiply them in a large amount, because they are absolute parasites and have no motility.

For multiplication of the microorganism of this type it has been common to employ a method wherein nematodes parasitized with such a microorganism are inoculated in the vicinity of the soil on which a crop plant is cultivated, followed by cultivation for a long period of time of at least two months, whereupon the multiplied microorganism is recovered from the root. However, the productivity is poor, and the method is hardly practically useful for actual agricultural production. In recent years, mass production of a natural enemy microorganism by means of sand has been reported by R.D. Sharma & G.R. Stirling (Nematologica vol. 37 No. 4 Oct. 1991 p483-484).

Further, it is possible that a plant root transformed by Agrobacterium or a simple root system is aseptically cultured on a culture medium such as an agar medium, and then nematode parasitized with the microorganism is aseptically inoculated thereto and multiplied under a controlled environment. However, the productivity is not better than the method using the soil.

Cultivation of upland crop plants in Japan is conducted usually in a field not suitable for a paddy field, because of leakage of water, such as a field of diluvial volcanic ash soil so-called black volcanic ash soil or alluvial sand loam or sand soil. It is also a well known fact that such soils are susceptible to damage by nematodes as compared with clay loam desired as paddy field soil. However, even with such soils suitable for upland crop plants, the efficiency is not still sufficient for mass production of the natural enemy microorganism. Namely, even if the nematode is applied from the soil surface together with irrigation water, or even if the nematode is mixed with the soil, it is impossible to let the nematode penetrate uniformly to the entire root system, and the penetration rate is insufficient.

The present inventors have found it possible to increase the number of the natural microorganism-deposited nematodes which penetrate into a plant root, by using an inorganic or organic carrier as the plant cultivation soil and selecting the particle size of the cultivation soil.

On the basis of this discovery, a study has been made for the productivity of the natural enemy microorganism, and it has been found that the natural enemy microorganism can certainly be produced efficiently by selecting the particle size of the carrier. The present invention was accomplished on the basis of these discoveries.

The present invention provides a method for efficiently producing a microorganism which is a natural enemy of nematodes, which comprises using an inorganic or organic carrier having a particle size of from 50 to 300 µm as a plant cultivation soil.

Namely, the natural enemy microorganism can be obtained in a large amount by inoculating the natural enemy microorganism-deposited nematode on the surface of the cultivation soil, followed by irrigation of water, or by adequately mixing such nematode with the cultivation soil, followed by cultivation under a proper temperature control.

The nematode useful in the present invention is preferably a root knot nematode or a cyst nematode, which is parasitic in plant roots. The plant may be any plant so long as such a nematode can be parasitic thereon.

The inorganic carrier to be used may, for example, be sea sand, river sand, mountain sand, silica sand or microballoons (water-floatable fine balls) obtained from the combustion residue of e.g. coal or cokes, or glass beads. As the organic carrier, coal powder may, for example, be mentioned.

With respect to the particle size, a material is preferred which contains at least 50% by weight of the inorganic or organic carrier having a particle size of from 50 to 300 µm.

The present invention will now be described with reference to Examples.

### EXAMPLE 1

### Penetrability of the natural enemy microorganism-deposited nematode into a plant root depending upon a difference in the particle size of the cultivation soil

A sample soil as identified in Table 1 was put in a clay pot, and seeds of tomato were sown. When twin leaves appeared after cultivation for nine days, 250 nematodes having natural microorganism Pasteuria spp. deposited thereon were applied from the soil surface to each plant.

After the application, a sufficient amount of water was supplied to disperse the nematodes in the soil. Then, the culture was left to stand at 20°C for 2 days. After the 2 days, the plant was recovered so that the root would not break and washed with 15% antiformin. Then, it was washed with water and immersed in an acidic Fuchsine solution and boiled. When cooled to an appropriate temperature, it was depigmented with acidic glycerol, and the number of nematodes penetrated into each sample root was counted. For each sample soil, the test was repeated four times. The results are shown in Table 1.

**Table 1**

| Sample soil | | Number of penetrated nematodes |
|---|---|---|
| Glass beads | No. 50 | 16.3 |
| | No. 70 | 29.8 |
| | No. 100 | 57.0 |
| | No. 150 | 44.5 |
| | No. 200 | 38.8 |
| | No. 400 | 0.3 |
| | No. 600 | 0.0 |
| | No. 800 | 0.0 |
| Sieved microballoons | 52-300 µm | 54.3 |
| Sieved river sand | 52-300 | 65.8 |

The particle sizes of the respective glass beads were as follows:
No. 50: 37-63 µm
No. 70: 63-88 µm
No. 100: 105-125 µm
No. 150: 149-177 µm
No. 200: 177-250 µm
No. 400: 350-500 µm
No. 600: 500-710 µm
No. 800: 710-990 µm

### EXAMPLE 2

### Production of the natural enemy microorganism by sand culture cultivation

To a suspension of the natural enemy microorganism Pasteuria spp., Meloidogyne incognita was added, and the suspension was left to stand overnight at 15°C in a test tube, so that about 10 natural enemy microorganisms were deposited per nematode. About 20,000 nematodes having the natural microorganisms deposited were inoculated to a pot surface of 1/10,000a where a tomato seedling was cultivated by means of black volcanic ash soil or sieved river sand as the cultivation soil. After the inoculation, water was supplied adequately to disperse the nematodes in the soil. Thereafter, cultivation was conducted at 25°C for 2 months. After the cultivation, the root was collected and homogenized, and the residue of the root was filtered off, and the produced natural enemy microorganisms were counted under a microscope. For each sample soil, the test was repeated three times. The results are shown in Table 2.

**Table 2**

| Sample soil | Number of produced microorganisms per seedling |
|---|---|
| Black volcanic ash soil | 3.1 × 10⁸ |
| Sieved river sand (52-300 µm) | 6.0 × 10⁹ |

According to the present invention, it has been made possible to efficiently produce the obligate parasite which is a natural enemy of plant parasitic nematode and which used to be difficult to produce.

## Claims

1. A method for efficiently producing a bacterium which is natural enemy to nematode, which comprises
(a) using an inorganic or organic carrier having a particle size of from 50 to 300 µm as a plant cultivation soil;
(b) inoculating nematode parasitized with said bacterium in the vicinity of crop plant in said soil;
(c) cultivating said crop plant for a period of time; and
(d) recovering the multiplied bacterium from the root of said crop plant.

2. A method according to Claim 1, which comprises using a material containing at least 50% by weight of the carrier as defined in Claim 1, as a plant cultivation soil.

## Patentansprüche

1. Verfahren zur effizienten Herstellung eines Bakteriums, das ein natürlicher Feind für einen Nematoden ist, welches umfaßt:
(a) eine Verwendung eines anorganischen oder organischen Trägers mit einer Teilchengröße von 50 bis 300 µm als Pflanzenkultivierungsboden;
(b) Inokulierung eines mit dem Bakterium parasitierten Nematoden in der Nähe einer Erntepflanze in dem Boden;
(c) Kultivierung der Erntepflanze für eine Zeitdauer; und
(d) Gewinnung des vermehrten Bakteriums aus der Wurzel der Erntepflanze.

2. Verfahren nach Anspruch 1, welches eine Verwendung eines Materials umfaßt, das mindestens 50 Gew.-% des Trägers, der in Anspruch 1 definiert wird, enthält, als Pflanzenkultivierungsboden.

## Revendications

1. Procédé pour produire efficacement une bactérie qui est un ennemi naturel des nématodes, qui comprend :
(a) l'utilisation d'un support organique ou inorganique ayant une grosseur de particules de 50 à 300 µm à titre de sol de culture des plantes ;
(b) l'inoculation des nématodes parasités avec ladite bactérie à proximité de la plante de culture dans ledit sol ;
(c) la culture de ladite plante de culture pendant un certain laps de temps ; et
(d) la récupération de la bactérie multipliée à partir de la racine de ladite plante de culture.

2. Procédé selon la revendication 1, qui comprend l'utilisation d'un matériau contenant au moins 50 % en poids du support tel que défini dans la revendication 1, à titre de sol de culture des plantes.
